# EUROPÄISCHE PATENTSCHRIFT

(11) **EP 0 918 047 B1**
(45) Veröffentlichungstag und Bekanntmachung des Hinweises auf die Patenterteilung: **10.04.2002**
(21) Anmeldenummer: 98119192.7
(22) Anmeldetag: 12.10.1998
(51) Int. Cl.: C07C 67/343, C07C 69/34, C07C 69/74

(54) **Verfahren zur C-Alkylierung von Malonsäureestern**
C-Alkylation process of esters of malonic acid
Procédé de C-alkylation d'esters de l'acide malonique

(30) Priorität: 24.11.1997 DE 19752041
(43) Veröffentlichungstag der Anmeldung: 26.05.1999
(73) Patentinhaber: Degussa AG, 40474 Düsseldorf (DE)
(72) Erfinder: Metz, Josef, Dr., 45770 Marl (DE); Osterholt, Clemens, 46286 Dorsten (DE)

(56) Entgegenhaltungen:
- DE-C- 4 326 917
- DATABASE WPI Week 9438 Derwent Publications Ltd., London, GB; AN 94-307604 XP002094327 & JP 06 234705 A (NIPPON SYNTHETIC CHEM IND CO), 23. August 1994

## Beschreibung

Die Erfindung betrifft ein Verfahren zur ein- oder zweifachen C-Alkylierung von Malonsäuredialkylestern mit Alkylhalogeniden oder Alkylendihalogeniden mit vicinalen Halogenatomen und Kaliumcarbonat (Pottasche) als Halogenwasserstoffakzeptor.

In DE 43 26 917 wird ein Verfahren zur Herstellung von Cyclopropan-1,1-dicarbonsäureestern aus Malonsäuredialkylestern und Alkylendihalogeniden mit vicinalen Halogenatomen (in der Folge kurz Alkylendihalogenide genannt) mit Kaliumcarbonat als Halogenwasserstoffakzeptor und Dimethylformamid oder Dimethylacetamid als Lösungsmittel beschrieben. Das Verfahren ist dadurch gekennzeichnet, daß man a) Alkylendichloride einsetzt, b) Kaliumcarbonat mit Feinkornanteilen unter 0,1 mm von gleich oder mehr als 85% und unter 0,05 mm von gleich oder mehr als 70% verwendet, c) während der Reaktion das Reaktionswasser azeotrop abdestilliert, d) die Reaktionstemperatur auf 90 bis 160°C bringt und e) das Molverhältnis von Dialkylmalonat zu Alkylendichlorid zu Kaliumcarbonat wie 1:(2,5 bis 3,5):(1,0 bis 1,4) wählt.

Dieses Verfahren stellt eine bedeutende Verbesserung gegenüber vorbekannten Arbeitsweisen dar. So erreicht man auch mit den wohlfeilen Alkylendichloriden Ausbeuten von über 80% d.Th., wie sie nach D.A.White, Synthetic Communications, 1977, S. 559 selbst mit den entsprechenden Alkylendibromiden nicht erzielt werden konnten. Statt des schwierig zu entsorgenden Kaliumbromids fällt das für die elektrolytische Herstellung von Kaliumhydroxid verwendbare Kaliumchlorid an. Die Raum-Zeit-Ausbeuten sind bei Reaktionszeiten von 5 bis 6 Stunden erheblich besser als bei dem Verfahren von D.A.White, loc.cit., das 22 Stunden benötigt, und bei der Arbeitsweise nach J.Heiszman et al., Synthesis Communications 1987, S. 738, bei der Alkylendichloride mit Benzol als Lösungsmittel eingesetzt werden, ein Phasentransferkatalysator verwendet wird und die Reaktionszeit 20 Stunden beträgt.

Es wurde nun gefunden, daß sich Malonsäuredialkylester vorteilhaft C-alkylieren lassen, wenn man Malonsäuredialkylester in Gegenwart von Kaliumcarbonat in einem inerten Lösemittel mit einem Alkylhalogenid oder einem Alkylendihalogenid umsetzt und dabei einen Phasentransferkatalysator erst dann zusetzt, wenn der Malonsäuredialkylester zu 50 bis 80% umgesetzt ist.

Setzt man ein Alkylhalogenid und einen unsubstituierten Malonsäuredialkylester ein, so erhält man, je nach dem Molverhältnis von Malonsäuredialkylester zu Alkylhalogenid zu Kaliumcarbonat das monooder das disubstituierte Derivat. Für die Herstellung von C-monosubstituierten Malonsäuredialkylestern wendet man die Stoffe vorteilhaft im Molverhältnis von 1:(1,5-3,0):(0,4-0,6), insbesondere von 1:(2,0-2,5):(0,45-0,5) an. Die Umsetzung verläuft nach der Gleichung 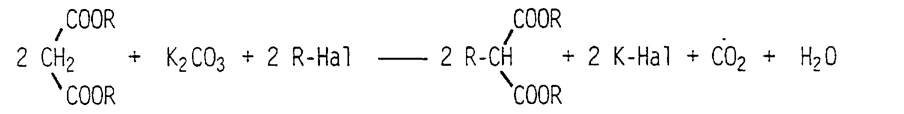 Dabei bedeutet R einen Alkylrest. Für die Herstellung von dialkylierten Derivaten wählt man vorteilhaft ein Molverhältnis von 1:(2,5-4,5):(1.0-1,5), insbesondere von 1:(2,5-3,0):(1,1-1,3). Ein Spezialfall der dialkylierten Derivate sind die Cyclopropanverbindungen, die man erhält, wenn man von einem Alkylendihalogenid ausgeht: 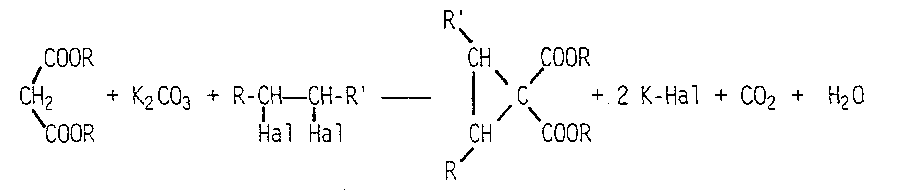 Dabei bedeuten R und R' jeweils unabhängig einen Alkylrest oder Wasserstoff. In diesem Falle ist ein Molverhältnis von 1:(2.5-3,5):(1,0-1,4), vorteilhaft von 1:(2,5-3,0):(1,0-1,4) empfehlenswert.

Mono-C-alkylierte Malonsäuredialkylester lassen sich nach dem erfindungsgemäßen Verfahren zu dialkylsubstituierten Derivaten umsetzen. Man kann auf diese Weise disubstituierte Malonsäuredialkylester mit verschiedenen Alkylsubstituenten herstellen.

Überraschenderweise lassen sich bei dem Verfahren nach der Erfindung die Reaktionszeiten erheblich - im Falle des erwähnten Verfahrens zur Herstellung von Cyclopropan-1,1-dicarbonsäureestern auf 3 Stunden - verkürzen und die Raum-Zeit-Ausbeuten entsprechend steigern.

Viele der als Ausgangsstoffe verwendeten Malonsäuredialkylester sind in kommerziellen Mengen erhältlich. Als Beispiele für geeignete Malonsäuredialkylester seien solche mit C₁₋₄-Alkylresten, wie Dimethylmalonat (DMM) und Diethylmalonat (DEM), die bevorzugt werden, sowie Diisopropylmalonat und Di-n-butylmalonat, genannt. Bevorzugte Alkylhalogenide bzw. Alkylendihalogenide sind die Chloride. Geeignete Alkylchloride und Alkylendichloride haben im allgemeinen bis zu 12, insbesondere bis zu 6 Kohlenstoffatome. Im einzelnen seien z.B. Methylchlorid (nur unter erhöhtem Druck umsetzbar), Ethylchlorid, Isopropylchlorid, Allylchlorid, Isoamylchlorid, 2-Chlorhexan, 1-Chloroctan, 1,2-Dichlorethan, 1,2-Dichlorpropan, 2,3-Dichlorbutan und 1,2-Dichlorcyclohexan erwähnt.

Das eingesetzte Kaliumcarbonat ist das übliche, kristallwasserfreie Produkt, das durch Mahlen, z.B. in Kugel- oder Stiftmühlen, auf feine Körnung gebracht wurde. Besonders geeignet ist ein Kaliumcarbonat mit Feinkornanteilen unter 0,1 mm von gleich oder mehr als 70% sowie unter 0,05 mm von gleich oder mehr als 85%.

Als inerte Lösungsmittel, die zweckmäßig mitverwendet werden, eignen sich besonders Dimethylformamid (DMF), Dimethylacetamid (DMA) und N-Methyl-2-pyrrolidon (NMP). Man wendet sie zweckmäßig in der 3- bis 4-fachen Gewichtsmenge an, bezogen auf das Gewicht des Malonsäuredialkylesters.

Das Reaktionswasser wird zweckmäßig mit Hilfe eines Schleppmittels aus dem Reaktionsgemisch entfernt. Wenn man dialkylierte Malonsäureester herstellt, kann man überschüssiges Alkylhalogenid oder Alkylendihalogenid als Schleppmittel benutzen. Dies ist auch möglich bei der Umsetzung von Alkylhalogeniden zu monoalkylierten Derivaten, indem man z.B. stöchiometrische Mengen Alkylhalogenid einsetzt und das mit dem Wasser abdestillierende Alkylhalogenid nach Kondensation und Phasentrennung in die Reaktion zurückführt. Alternativ kann man auch eines der üblichen inerten Schleppmittel zusetzen, z.B. Toluol.

Es ist ein wichtiges Merkmal des Verfahrens nach der Erfindung, daß man die Reaktion bis zu einem Umsatz des Malonsäuredialkylesters von 50 bis 80% ablaufen läßt und dann einen Phasentransferkatalysator zusetzt und die Reaktion zu Ende führt. Der Phasentransferkatalysator erleichtert die Wechselwirkung zwischen den in flüssiger Phase vorliegenden Reaktanten mit dem festen Kaliumcarbonat, wie aus der vermehrten Kohlendioxidentwicklung ersichtlich ist, die durch den Zusatz des Phasentransferkatalysators ausgelöst wird. Geeignete Phasentransferkatalysatoren sind z.B. quartäre Ammoniumsalze, wie Tetraalkylammoniumsalze, vorteilhaft mit C₁- bis C₈- und insbesondere mit C₁ bis C₄-Alkylresten, sowie die entsprechenden Ammoniumhydroxide, wie Tetra-n-butylammoniumhydroxid. Bei den Salzen kann es sich z.B. um Halogenide, Hydrogensulfate oder Sulfate handeln. Als Vertreter dieser Stoffklassen seien z.B. Tetrabutylammoniumbromid (TBAB), Tetrabutylammoniumhydrogensulfat, Tetraoctylammoniumbromid, Benzyltriethylammoniumchlorid und Methyltrioctylammoniumchlorid genannt. Weiterhin geeignet sind die entsprechenden Phosphoniumverbindungen, wie Tetra-n-butylphosphoniumhydroxid oder -bromid. Andere in der vorliegenden Erfindung verwendbare Phasentransferkatalysatoren sind Kronenether, insbesondere 18-Krone-6. Man wendet die Phasentransferkatalysatoren zweckmäßig in der 0,003- bis 0.01-fachen Gewichtsmenge an, bezogen auf das Gewicht des Malonsäuredialkylesters. Bei diesen geringen Mengen bereitet die Entsorgung keine besonderen Probleme, und es fallen selbst bei den höheren Temperaturen während der Aufarbeitung, bei denen Zersetzung eintreten könnte. keine nennenswerten Mengen an Aminen an.

Zur Durchführung des Verfahrens nach der Erfindung kann man die organischen Reaktanten, das Kaliumcarbonat sowie das Lösungsmittel und gegebenenfalls ein inertes Schleppmittel in einem Reaktionsgefäß vorlegen und das Gemisch, zweckmäßig unter Rühren, auf die Reaktionstemperatur erhitzen. Man kondensiert das abdestillierende Azeotrop, läßt die Phasen sich trennen, zieht die Wässrige Phase ab und führt die organische Phase in das Reaktionsgefäß zurück. Man verfolgt das Fortschreiten der Reaktion über Abgasmessungen und GC-Analysen des Reaktionsgemisches.

Sobald die Reaktion nachläßt und 50 bis 80%, vorteilhaft 60 bis 70 % des Malonsäuredialkylesters umgesetzt sind, setzt man, zweckmäßig nach und nach innerhalb von 15 bis 45 Minuten, den Phasentransferkatalysator zu, worauf die Reaktion wieder anspringt, erkennbar an der lebhaften Kohlendioxidentwicklung. Der Phasentransferkatalysator kann als wäßrige Lösung zugeführt werden. Nach insgesamt 3 bis 17 Stunden ist die Umsetzung praktisch beendet. Auf diese Weise läßt sich z.B. bei der Herstellung des Cyclopropan-1,1-dicarbonsäuredimethylesters ein Umsatz des Malonsäuredialkylesters von >98% erreichen. Setzt man den Phasentransferkatalysator schon zu Beginn der Reaktion zu, so erzielt man unter sonst gleichen Bedingungen nur einen Umsatz von 93%, und das überschüssige Kaliumcarbonat wird überwiegend zur intramolekularen Dehydrochlorierung der Halogenverbindung unter Bildung von olefinisch ungesättigten Verbindungen verbraucht.

Die Verfahrensprodukte sind bekannte Stoffe und wertvolle Zwischenprodukte für weitere Synthesen, z.B. auf dem Pharma- und Pflanzenschutzgebiet.

Die folgenden Beispiele sollen die Erfindung weiter erläutern, nicht aber ihren Anwendungsbereich begrenzen, wie er in den beigefügten Patentansprüchen definiert ist.

### Beispiel 1 - Cyclopropan-1,1-dicarbonsäuredimethylester

In einem Reaktionsgefäß mit Rührer und Destillationsaufsatz mit Kondensator und Phasentrenngefäß werden 2,1 mol DMM, 6,6 mol EDC, 2,4 mol Pottasche mit 87 Gew.-% Anteilen mit Korngrößen von <0,1 mm und 78 Gew.-% Anteilen mit Korngrößen von < 0,05 mm sowie 6,5 mol DMF vorgelegt. Das Gemisch wird unter Rühren auf 110°C erhitzt, wobei Reaktionswasser mit EDC azeotrop abdestilliert. Das Reaktionswasser wird als obere Phase abgezogen, das EDC als untere Phase in die Reaktion zurückgeführt.

Nach 1,5 h sind 65% des DMM umgesetzt. Man setzt innerhalb von 30 min 0,008 mol TBAB als 50 gewichtsprozentige wäßrige Lösung zu. Dabei steigt die Kohlendioxidentwicklung wieder an. Nach einer weiteren Stunde, also nach 3 h ab Reaktionsbeginn, wird ein DMM-Umsatz von ca. 98% erreicht. Die Reaktion wird dann beendet; die Umsetzung des restlichen DMM findet in der nun folgenden Aufarbeitungsstufe statt.

Vom Reaktionsgemisch wird zunächst bei einer Temperatur von 110 bis 120°C überschüssiges EDC abdestilliert, wobei der DMM-Umsatz auf >99% ansteigt. Anschließend werden mit einem Rotationsverdampfer (160°C; 1 mbar) flüchtige Anteile von Hochsiedern und Salz abgetrennt. Das Destillat, überwiegend DMF und das Zielprodukt Cyclopropan-1,1-dicarbonsäuredimethylester, wird fraktioniert. Man erhält das Zielprodukt in einer Reinheit von 99,3 % (GC-Analyse) und mit einer Ausbeute von ca. 83 % d.Th., bezogen auf eingesetztes DMM.

Das aus der Reaktion entweichende Abgas (überwiegend CO₂ und wenig H₂O, VC und EDC) wird einer CKW-Verbrennung zugeführt. Die bei der destillativen Aufarbeitung des Reaktionsansatzes anfallenden Wertstoffe EDC und DMF können für einen neuen Ansatz verwendet werden.

### Vergleichsbeispiel 1

Man verfährt wie im Beispiel 1, setzt jedoch den Phasentransferkatalysator bereits zu Beginn zu. Es wird unter sonst gleichen Bedingungen mehr Pottasche für die Bildung von Vinylchlorid verbraucht. Nach 3 Stunden war kaum noch Pottasche vorhanden, das DMM jedoch nur zu 93 % umgesetzt.

### Beispiel 2 - n-Butylmalonsäurediethylester

6,0 mol DMF, 2,5 mol n-Butylchlorid (n-BuCl). 0,95 mol Kaliumcarbonat gemäß Beispiel 1 und 2 mol Malonsäurediethylester(DEM) werden vorgelegt. Das Gemisch wird unter Rühren auf 110-120°C erhitzt, wobei das Reaktionswasser mit n-BuCl azeotrop abdestilliert. Das Reaktionswasser wird nach Kondensation des Azeotrops als untere Phase abgezogen, das n-BuCl als obere Phase zurückgeführt. Um eine genügend große Destillationsleistung aufrechtzuerhalten, werden während der Reaktion weitere 2 mol n-BuCl nachdosiert. Nach einer Reaktionszeit von 2 Stunden werden 0,008 mol TBAB als 50 gewichtsprozentige wässerige Lösung innerhalb von 30 min zudosiert.

Nach einer Gesamtreaktionszeit von 6h wird eine 92%iger DEM-Umsatz erreicht, bestimmt durch GC-Analyse. Bei der Abtrennung des restlichen n-BuCl steigt der Umsatz auf 93,5% an. Die weitere Aufarbeitung erfolgt analog dem Beispiel 1. Man erhält das Zielprodukt in einer Reinheit von >99% (GC-Analyse), die Ausbeute beträgt 85% der Theorie, bezogen auf den DEM-Umsatz. Die bei der destillativen Aufarbeitung des Reaktionsansatzes anfallenden Wertstoffe n-BuCl, DEM und DMF können für einen neuen Ansatz verwendet werden.

### Beispiel 3 - Di-n-propylmalonsäurediethylester

6 mol NMP, 1,2 mol n-Propylchlorid (n-PrCl), 2,4 mol Kaliumcarbonat gemäß Beispiel 1 und 2 mol DEM werden vorgelegt. Die Reaktion wird analog dem Beispiel 2 über eine Gesamtreaktionszeit von 17 Stunden geführt. Nach 2h und 7 h werden jeweils 0,008 mol TBAB als 50 gewichtsprozentige wässerige Lösung zudosiert. Innerhalb der Gesamtreaktionszeit werden 4,8 mol n-PrCl nachdosiert.

Man erhält das Zielprodukt in einer Reinheit von >99% (GC-Analyse). die Ausbeute beträgt ca. 78 % der Theorie, bezogen auf den DEM-Einsatz. Die bei der destillativen Aufarbeitung des Reaktionsansatzes anfallenden Wertstoffe n-PrCl, Mono-n-propylmalonsäurediethylester und NMP können für einen neuen Ansatz verwendet werden.

### Beispiel 4 - Ethylmalonsäurediethylester

6 mol DMF, 0,95 mol Kaliumcarbonat gemäß Beispiel 1 und 2 mol DEM werden vorgelegt. Die Reaktion wird bei 110-120°C analog dem Beispiel 2 über eine Gesamtreaktionszeit von 6h geführt. Während dieser Zeit werden 5 mol 1-Chlorethan eingeleitet. Der Kondensator wird mit einem Kryostat auf ca. 1°C eingestellt, die obere Phase des kondensierten Azeotrops besteht aus 1 Chlorethan und wird in die Reaktion zurückgeführt. Nach einer Reaktionszeit von 2,5 h wird der Phasentransferkatalysator (0,008 mol TBAB als 50 gewichtsprozentige wässerige Lösung) zugesetzt.

Der DEM-Umsatz beträgt nach 6h ca. 98% der Theorie. Nach der destillativen Aufarbeitung des Reaktionsgemisches erhält man das Zielprodukt in einer Reinheit von >99% (GC-Analyse). Die Ausbeute beträgt ca. 86% d.Th., bezogen auf den DEM-Umsatz.

## Patentansprüche

1. Verfahren zur C-Alkylierung von Malonsäuredialkylestern, **dadurch gekennzeichnet, daß** man Malonsäuredialkylester in Gegenwart von Kaliumcarbonat als Halogenwasserstoffakzeptor in einem inerten Lösungsmittel mit einem Alkylhalogenid oder einem 1,2-Alkylendihalogenid umsetzt und dabei einen Phasentransferkatalysator erst dann zusetzt, wenn der Malonsäuredialkylester zu 50 bis 80% umgesetzt ist.

2. Verfahren nach Anspruch 1, **dadurch gekennzeichnet, daß** der Malonsäuredialkylester Dimethylmalonat oder Diethylmalonat ist.

3. Verfahren nach Anspruch 1 oder 2, **dadurch gekennzeichnet, daß** man Kaliumcarbonat mit Feinkornanteilen unter 0,1 mm von gleich oder mehr als 85 Gew.-% und unter 0,05 mm von gleich oder mehr als 70 Gew.-% verwendet.

4. Verfahren nach einem der Ansprüche 1 bis 3, **dadurch gekennzeichnet, daß** die Alkylhalogenide und die 1,2-Alkylendihalogenide Chloride sind.

5. Verfahren nach einem der Ansprüche 1 bis 4, **dadurch gekennzeichnet, daß** der Phasentransferkatalysator ein Tetraalkylammoniumsalz oder -hydroxid, ein Tetraalkylphosphomiumsalz oder - hydroxid oder ein Kronenether ist.

6. Verfahren nach Anspruch 5, **dadurch gekennzeichnet, daß** der Phasentransferkatalysator Tetra-n-butylammoniumbromid, Tetra-n-butylammoniumhydrogensulfat, Tetra-n-butylphosphoniumbromid oder 18-Krone-6 ist.

7. Verfahren nach einem der Ansprüche 1 bis 6, **dadurch gekennzeichnet, daß** man als inertes Lösungsmittel Dimethylformamid, Dimethylacetamid oder N-Methyl-2-pyrrolidon verwendet.

8. Verfahren nach einem der Ansprüche 1 bis 7, **dadurch gekennzeichnet, daß** man die Umsetzung bei Temperaturen von 90 bis 160°C unter azeotropem Abdestillieren des Reaktionswassers durchführt.

## Claims

1. A process for the C-alkylation of a dialkyl malonate, **characterized in that** a dialkyl malonate is reacted with an alkyl halide or a 1,2-alkylene dihalide in the presence of potassium carbonate as a hydrogen halide acceptor in an inert solvent, and a phase-transfer catalyst is not added until from 50 to 80% of dialkyl malonate has reacted.

2. A process according to claim 1, **characterized in that** the dialkyl malonate is dimethyl malonate or diethyl malonate.

3. A process according to claim 1 or 2, **characterized in that** potassium carbonate having fine particle fractions of 85% by weight or more smaller than 0.1 mm and 70% by weight or more smaller than 0.05 mm is used.

4. A process according to any one of claims 1 to 3, **characterized in that** the alkyl halide and the 1,2-alkylene dihalide are chlorides.

5. A process according to any one of claims 1 to 4, **characterized in that** the phase-transfer catalyst is a tetraalkylammonium salt or a tetraalkylammonium hydroxide, a tetraalkylphosphonium salt or a tetraalkylphosphonium hydroxide or a crown ether.

6. A process according to claim 5, **characterized in that** the phase-transfer catalyst is tetra-n-butylammonium bromide, tetra-n-butylammonium hydrogen sulphate, tetra-n-butylphosphonium bromide or 18-crown-6.

7. A process according to any one of claims 1 to 6, **characterized in that** the inert solvent used is dimethylformamide, dimethylacetamide or N-methyl-2-pyrrolidone.

8. A process according to any one of claims 1 to 7, **characterized in that** the reaction is carried out at temperatures of from 90 to 160°C with removal of the water of reaction by azeotropic distillation.

## Revendications

1. Procédé d'alkylation au carbone d'esters dialkyliques d'acide malonique,
caracténsé en ce qu'
on fait réagir un ester dialkylique d'acide malonique en présence de carbonate de potassium en tant qu'accepteur d'acide halohydrique, avec un halogénure d'alkyle ou avec un halogénure de 1,2-dialkylène dans un solvant inerte, et pour cela on ajoute un catalyseur de transfert de phase seulement après que l'ester dialkylique d'acide malonique est transformé pour 50 à 80 %.

2. Procédé selon la revendication 1,
**caractérisé en ce que**
l'ester dialkylique d'acide malonique est le malonate de diméthyle ou le malonate de diéthyle.

3. Procédé selon Tune quelconque des revendications 1 ou 2,
**caractérisé en ce qu'**
on utilise du carbonate de potassium ayant des fractions à grains fins avec un pourcentage égal ou supérieur à 85 % en paids en dessous de 0,1 mm, et égal ou supérieur à 70 % en poids en dessous de 0,05 mm.

4. Procédé selon l'une quelconque des revendications 1 à 3,
**caractérisé en ce que**
les halogénures d'alkyle et les halogénures de 1,2- alkylène sont des chlorures.

5. Procédé selon l'une quelconque des revendications 1 à 4,
**caractérisé en ce que**
le catalyseur de transfert de phase est un sel ou un hydroxyde de tétraalkylammonium, un sel ou un hydroxyde de tetraalkylphosphonium ou un éther couronne.

6. Procédé selon la revendication 5,
**caractérisé en ce que**
le catalyseur de transfert de phase est le bromure de tétra-n-butylammonium, l'hydrogénosulfate de tétra-n-butylammonium, le bromure de tétra-n-butylphosphonium ou l'éther 18-couronne-6.

7. Procédé selon Tune quelconque des revendications 1 à 6,
**caractérisé en ce qu'**
on utilise comme solvant inerte du diméthylformamide, du diméthylacétamide ou de la N-méthyl-2-pyrrolidone.

8. Procédé selon Tune quelconque des revendications 1 à 7,
**caractérisé en ce qu'**
on effectue la réaction à des températures allant de 90 à 160°C tout en séparant par distillation azéotropique l'eau de réaction.
